Europäisches Patentamt

**European Patent Office**

**Office européen des brevets**

(19)

(11) Publication number: **0 182 278**
A2

(12) # EUROPEAN PATENT APPLICATION

(21) Application number: **85114449.3**

(22) Date of filing: **13.11.85**

(51) Int. Cl.⁴: **C 07 K 7/10,** C 07 K 1/14,
A 61 K 37/02, A 23 K 1/17

(30) Priority: **19.11.84 JP 244162/84**

(43) Date of publication of application: **28.05.86**
**Bulletin 86/22**

(84) Designated Contracting States: **DE FR GB**

(71) Applicant: **WAKUNAGA SEIYAKU KABUSHIKI KAISHA,**
**1-39 Fukushima 3-chome Fukushima-ku, Osaka-shi**
**Osaka-fu (JP)**

(72) Inventor: **Natori, Shunji, 2208-19, Fukawa Tone-Machi,**
**Kitasoma-Gun Ibaraki-Ken (JP)**

(74) Representative: **Kraus, Walter, Dr. et al, Patentanwälte**
**Kraus, Weisert & Partner Thomas-Wimmer-Ring 15,**
**D-8000 München 22 (DE)**

(54) **Antibacterial polypeptide, preparation thereof, and use thereof.**

(57) An antibacterial polypeptide is disclosed, which is represented by the amino acid sequence:

$H_2N$-Gly-Trp-Leu-$X_1$-Lys-Ile-Gly-Lys-Lys-Ile-Glu-Arg-Val-Gly-Gln-His-Thr-Arg-Asp-Ala-Thr-Ile-Gln-$X_2$-$X_3$-Gly-$X_4$-Ala-Gln-Gln-Ala-Ala-Asn-Val-Ala-Ala-Thr-Ala-Arg-COOH

wherein: $X_1$ represents Lys or Arg; $X_2$, Gly or Val; $X_3$, Leu or Ile; and $X_4$, Ile or Val, and which is produced by imparting injury to the body wall of larvae of an insect belonging to the order Diptera or Hymenoptera such as Sarcophaga peregrina, obtaining the hemolymph from the larvae thus injured, removing hemocytes and fatty components from said hemolymph, and then obtaining, from the thus purified hemolymph, the polypeptide. The polypeptide is characterized by its antibacterial activity against a lot of Gram (–) bacteria and by being edible.

1

ANTIBACTERIAL POLYPEPTIDE,

PREPARATION THEREOF, AND USE THEREOF

BACKGROUND OF THE INVENTION

Field of the Art

This invention relates to a novel antibacterial polypeptide, preparation thereof, and use thereof.

Prior Art

There have been some reports[1,2,3,4] that antibacterial substances appear in the hemolymph by inoculation of vaccines in invertebrates such as insects. Such substances may be considered to bear important roles for biophylaxis of animals that have no immune network, but their induction mechanisms, properties of the substances, mechanisms of antibacterial actions, and other characteristics have not generally been amply clarified so far.

We have already clarified[5] that, in the case of Sarcophaga peregrina larva, when its body wall is injured, proteins having marked antibacterial activity against E. coli or Bacillus subtilis appear in its hemolymph. This may be considered to be a kind of biophylactic substance which is induced for prevention of bacterial infection during injury of the body wall. In this insect, also during injury of its body wall, proteins with hemagglutinating activity having the ability of activating phagocyte have been found to appear[6,7], and therefore these proteins seem to be related to each other to constitute the biophylactic mechanism for Sarcophaga peregrina. On the other hand, also in higher mammals, the presence of antibacterial proteins having potent activities and broad spectra has been recently found in the semen[8] or serum[9]. Thus, antibacterial proteins in the hemolymph of animals in general are attracting attention as matters of great significance.

Concerning the antibacterial proteins of Sarcophaga peregrina, we have further reported three kinds of proteins[10], were successful in purifying one component of these to a protein, which is electrophoretically single and elevated in antibacterial specific activity by 10,000-fold or more, particularly approximately 30,000-fold or more, and have also clarified a part of the physicochemical properties thereof (Japanese Patent Laid-Open Publication No.13730/1984, and 102nd Annual Meeting of Pharmacological Society of Japan and its abstracts of lectures).

However, it has recently been found that this component (the "third component" in the above Japanese Patent Laid-Open Publication No.13730/1984) is not yet a single protein but consists of plural components. Accordingly, also as viewed from the standpoint of, for example, medicine or pharmacology, utilization of this component is insufficient, and complete identification of the plural components has been desired.

SUMMARY OF THE INVENTION

The present invention is based on our successful isolation and identification of a plurality of single proteins obtained from one component, specifically the third component, induced upon injury of the body wall of a larva of an insect belonging to Diptera or Hymenoptera, above all Sarcophaga peregrina, by further applying an operation for purification of this component.

The antibacterial polypeptide according to the present invention is represented by the following amino acid sequence:

$H_2N$-Gly-Trp-Leu-$X_1$-Lys-Ile-Gly-Lys-
Lys-Ile-Glu-Arg-Val-Gly-Gln-His-
Thr-Arg-Asp-Ala-Thr-Ile-Gln-$X_2$-$X_3$
-Gly-$X_4$-Ala-Gln-Gln-Ala-Ala-Asn-
Val-Ala-Ala-Thr-Ala-Arg-COOH

wherein $X_1$ represents Lys or Arg, $X_2$ Gly or Val, $X_3$ leu or Ile, and $X_4$ Ile or Val.

Also, the process for producing the antibacterial polypeptide according to the present invention comprises imparting injury to the body wall of a larva of an insect belonging to Diptera or Hymenoptera and then obtaining a polypeptide represented by the above amino acid sequence formula from the body fluid obtained, from which hemocytes and fatty components have been removed.

Further, the edible antibacterial agent according to the present invention comprises the polypeptide represented by the above amino acid sequence formula as the effective ingredient.

The antibacterial polypeptide according to the present invention itself has antibacterial property and is substantially free from toxicity. Therefore, various uses utilizing these characteristics may be considered. Particularly, application of these substances for pharmaceutical preparations comprising these as effective components or utilization as additives in foods may be expected.

Above all, the polypeptide obtained from the larva of Sarcophaga peregrina is thermally stable, and it can be expected to be applied in uses including processing steps with heat.

BRIEF DESCRIPTION OF THE DRAWING

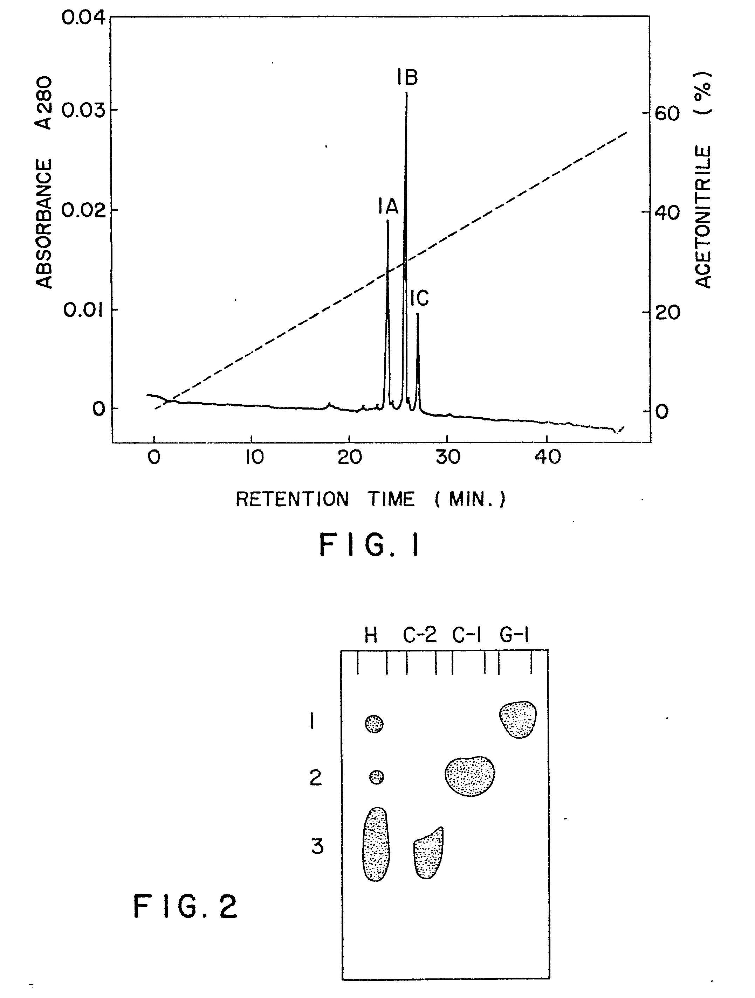

In the drawing, Fig. 1 is a chart showing the elution pattern of sarcotoxin I by high performance liquid chromatography. Fig. 2 shows the result of polyacrylamide gel electrophoresis.

DETAILED DESCRIPTION OF THE INVENTION

1. Subject insect and obtaining of body fluid

The insect to be used in the present invention is an insect belonging to the order Diptera or Hymenoptera. Specifically, a fly, mosquito, bee, horsefly, etc. are included. Among these, a fly of the family of Sarcophaga (Sarcophagidae), particularly Sarcophaga peregrina is preferred.

4

The subject insect must be its larva. Here, "larva" refers to the insect after hatching and before pupation in the process of complete metamorphosis of an insect. The larva suitable for use in the present invention is a third-instar larva, particularly Sarchophaga peregrina larva.

The operation to impart injury to the body surface of an insect so as to induce the antibacterial peptide may be practiced according to any desired method which can increase consequently the danger of infection. The "body wall" means not only the surface of the body but any part of the body which can be injured by an operation such as penetration of an injection syringe. The body wall to which an injury is to be imparted may be any desired portion of the insect, but generally it is a portion other than the head portion.

The insect's hemolymph is squeezed out 0 to 5 days, desirably about 2 days, after the injury, and then, hemocytes are removed by centrifugation. The resultant supernatant is heated in a hot water bath (e.g., at 100°C) for 5 minutes to remove further fatty components and unnecessary proteins therefrom. After centrifugation, the supernatant is recovered and used as the starting material.

2. Purification and separation of antibacterial polypeptide

Various methods conventionally used for fractionation and purification of macromolecular proteins in general are applicable. However, it is desirable to proceed with the purification of the material in a state wherein it is containing all of the active components and to carry out separation of the respective components (among them, one is the objective component of the present invention) when the impurities have decreased in amount. In the present invention, particularly when employing Sarcophaga peregrina larva, it is preferable to use a method in which purification is accomplished by the two purification procedures of ion-exchange resin treatment and heat treatment while all of the active components are contained within the process material, and concentration is carried out by ultrafiltration, the respective components then being separated by gel filtration and ion-exchange resin treatment. Furthermore, this kind of antibacterial substance is clearly a proteinous substance since it is deactivated by trypsin treatment. Hence, it is preferable to use increase of specific activity per protein as an index in performing the above purification and to use the fact that there is no lowering in specific activity of the respective active components as a whole as an index in performing the above separation.

After separation of the respective components, when one component thereof is further purified to the single substance of the present invention, various methods are conceivable, as described above. For example, the purification can be accomplished by using suitably an ion-exchange resin treatment, adsorption chromatography, electrophoresis, etc. However, it is preferable to carry out the operation by using as an index the satisfying of the two conditions, namely, that single bands should be formed according to two electrophoretic systems of different principles and that the activities should correspond to the respective bands, as described in an Example of the present invention.

Also, since the antibacterial protein has good heat resistance, the crude preparation can be heated for purification and for enhancement of specific activity. That is, it is convenient to subject the crude preparation to heat treatment at 80 to 120°C, preferably around 100°C, for about 10 to 60 minutes.

3. Antibacterial polypeptide

The antibacterial polypeptide according to the present invention is induced in a the hemolymph of larvae by stimulation in the form of a body wall injury. This can be confirmed by comparison between hemolymph to which a body wall injury has been imparted and the normal hemolymph as to whether a band inhibited in colony formation of bacteria appears or not when the hemolymph of larvae is subjected to, for example, electrophoresis and left to stand with spraying of E. coli, etc. thereon.

Also, as mentioned above, the substance of this band can be confirmed to be a proteinous substance by deactivation by, for example, trypsin treatment.

The antibacterial polypeptide according to the present invention is represented by the aforegiven amino acid sequence formula.

The amino acid sequence formula was determined by means of a Protein Sequencer (produced by Applied Biosystems Co.).

The antibacterial polypeptide is also soluble in aqueous methanol.

The antibacterial polypeptide can also be freeze-dried to obtain a white powder.

4. Use

The antibacterial polypeptide according to the present invention is useful as an antibacterial agent by utilizing its antibacterial property. That is, it can be used as an antibacterial agent as it is or in the form combined with an appropriate liquid, solid or gaseous carrier or diluent, or in the form combined with other drugs. As can be seen from the Laid-Open Patent Publication supra, the antibacterial polypeptide of the present invention has a broad antibacterial spectrum, clearly exhibiting antibacterial action against both Gram-negative and Gram-positive bacteria.

Accordingly, the antibacterial polypeptide of the present invention can be used as a drug against, for example, bacterial diseases. In that case, the type of preparation for administration and its dosage may be chosen as desired, provided that the antibacterial effect by the present invention can be recognized in consideration of the patient, the kind of disease, symptoms, etc. While it is important for such a drug that its activity should not be lost in blood serum, the antibacterial polypeptide of the present invention is valuable in that its antibacterial activity can be recognized in the presence of serum, as is also apparent from the Laid-Open Patent Publication supra.

The antibacterial polypeptide of the present invention is a protein per se, and may be considered to be substantially free from any toxicity at least consequently when taken up orally. Accordingly, the antibacterial polypeptide can be utilized as a drug and an additive in foods or fodders for human beings and animals. For example, the antibacterial polypeptide according to the present invention is useful as an antibacterial agent as an additive in foods, in other words, an edible antibacterial agent.

5. Experimental Examples

1) Materials for experiment and measuring methods:

The materials and the methods employed in this experiment are as described below.

(1) Starting material:

As Sarcophaga peregrina larva, a third-instar larva so prepared by inhibiting puparium formation by wetting the body surface with water according to the method of Ohtaki et al was used.

The operation of imparting an injury onto the body wall was performed by immobilizing the third-instar larvae by placing it on ice, placing this on a glass plate, and penetrating the lower part of the first segment of the larva with a Terumo injection syringe (SB. 26Gx1/2"). Two days after the imparting of the injury, the head portion of the larva was cut off with surgical scissors, and the hemolymph was squeezed out by pressing the larva against the inner wall of a funnel made of glass ($\phi$ 9 cm) cooled on ice. This was collected in a Spitz tube, subjected to centrifugation at 3,000 rpm (5 minutes, 4°C, Kokusan 103R), for removing hemocytes, and its supernatant was stored at -80°C as the hemolymph preparation. For obtaining a purified material from this preparation, the stored preparation was melted in a water bath at 37°C and subjected to centrifugation at 10,000 g (10 minutes, 4°C, Kubota RA-2) for further removing fatty components mixed therein, and the supernatant was collected.

(2) Buffers:

The buffers used had the compositions as shown below:

| Buffer | $Na_2HPO_4/NaH_2PO_4$ (pH 6.0) | NaCl |
|---|---|---|
| A | 10 mM | 0 mM |
| B | " | 25 mM |
| S | " | 130 mM |
| D | " | 260 mM |
| E | " | 520 mM |
| | $Na_2HPO_4/NaH_2PO_4$ (pH 7.0) | |
| F | 200 mM | |

(3) Antibacterial activity unit:

The activity quantity which inhibits 50% of the growth after shaking cultivation of $10^7$ cells of E. coli (K-12 $SM^r$) at 37°C for 140 minutes in 2 ml of a culture medium [1 ml of Buffer S containing 0.2% BSA, 1 ml of 1.75% Antibiotic Medium 3 (Difco Co.) (hereinafter called AM3 culture medium)] is defined as one unit.

(4) Method for measurement of antibacterial activity:

E. coli K-12 594 (Streptomycin-resistant) was used for measuring of antibacterial activity. After shaking culture was carried out in AM3 culture medium (1.75%, Monod test tube) at 37°C to the logarithmic proliferation phase, the culture broth was cooled in ice, centrifuged at 10,000 g (10 min., 4°C, Kubota RA-2) to remove the culture medium, and diluted with

Buffer S to $OD_{650}$ of ca. 0.3. The E. coli suspension of this concentration was calculated to be $2.5 \times 10^8$ cells/ml, as counted by means of a phase contrast microscope.

Subsequently, 10 μl of the E. coli suspension, 190 μl of AM3 culture medium, and 200 μl of the sample to be measured were mixed, cultured at 37°C for 140 minutes, then ice-cooled to stop the growth of the bacteria, and $OD_{650}$ was measured. In this measuring system, $OD_{650}$ of the sample to be measured was represented in terms of % relative to Control. Therefore, according to this measurement method, the growth inhibitory activity as the sum of sterilizing action and controlling action of the sample is measured.

(5) Method for preparation of a column for purification:

(a) CM-cellulose and hydroxylapatite:

After a powder of each of the material is suspended in deionized water, fine particles are removed by decantation (x 3). After washing by replacement with the buffer to be used, the powder is packed in a column, and washed with 10-fold or more volume of the column volume of the buffer in the case of CM-cellulose and 20-fold or more volume of the column volume of the buffer in the case of hydroxylapatite.

(b) Sephadex G-50:

After a powder of the material is suspended in deionized water, fine particles are removed by decantation (x 3). After washing by replacement with Buffer S, the suspension is left to stand on a boiling water bath for 3 hours for swelling. After packing in the column, washing with 5-fold or more of the column volume of Buffer S is carried out. The column, after passing of the sample, is washed with Buffer S and provided for re-use.

(6) Electrophoretic method:

(a) 15% polyacrylamide slab gels electrophoresis under non-denaturing conditions:

After 25 ml of Solution A (4.8 ml of 1N potassium hydroxide, 17.2 ml of acetic acid and 4.0 ml of N,N,N,N'-tetramethylethylenediamine made up to 100 ml with addition of water), 5 ml of Solution B (60 g of acrylamide and 0.8 g of N,N'-methylenebisacrylamide made up to 100 ml with addition of water), 10 ml of Solution C (0.28 g of ammonium persulfate made up to 100 ml with addition of water) and 2.5 ml of water were mixed, the mixture was promptly degassed and injected into a slab electrophoretic device. Next, a comb for applying was inserted, and gelation was carried out by leaving the mixture to stand at room temperature for 40 minutes. To 100 μl of the sample were added 50 μl of acetic acid (1M) containing 5 μg/ml of Methyl Green and 20 μl of 50% glycerol, and 10 to 50 μl of this mixture was applied to the track wherefrom the comb had been withdrawn, and electrophoresis was carried out at 200 V for 6 hours. As the electrophoretic buffer, a mixture of 3.12 g of β-alanine and 0.8 ml of acetic acid added into water to make up one liter was used.

The antibacterial active band was detected according to the following method.

After completion of electrophoresis, the gels were removed from the glass plate, soaked in 1.75% of AM3 culture medium containing 10 μg/ml of Streptomycin and Buffer F, and warmed at 37°C for 30 minutes. Then, the gels were spread on a glass laboratory dish, and immediately 0.6% nutrient agar (soft agar) containing $10^5$ cells of E. coli (K-12SM$^r$) and Buffer F was overlaid thereon. The soft agar had previously been heated to 47°C to be liquefied and solidified after overlaying at 37°C. On this layer was overlaid the same agar containing no E. coli and left to stand at 37°C overnight. Then, with a black paper being placed beneath the laboratory dish and with irradiation with light from the side to illuminate the colony of E. coli formed, the spot at which colony formation was inhibited was observed.

(b)   SDS-polyacrylamide gel electrophoresis (slab, 15%):
Following the method of Laemmli et al[11], a gel with 15% polyacrylamide gel concentration was used.

Staining and decolorization were performed following the methods of Fairbanks et al[12]. However, since the antibacterial protein dealt with in the present research cannot be readily immobilized on the gel, the time required for staining and decolorization was made not to exceed 10 hours under the condition of heating at 37°C.

(7)   Protein quantitation:

Protein quantitation was performed according to a modified Lowry method[13]. That is, to an appropriate amount of a sample (5 - 20 μg) was added a buffer to adjust finally the concentration to 1/2 salt concentration of Buffer S.  To 200 μl of the resultant solution was added 200 μl of 10% trichloroacetic acid, and the mixture was heated at 70°C for 20 minutes and left to stand in ice for 30 minutes or longer.  The precipitates formed by centrifugation at 3,000 rpm (15 minutes, 4°C, Kokusan 103 R) were recovered, mixed with 200 μl of Solution C for the Lowry method and 200 μl of deionized water, left to stand at room temperature for 10 minutes, supplied with 20 μl of phenol reagent, heated at 37°C for 40 minutes, and thereafter $OD_{700}$ was measured with a Kubota 150-02 photometer.  Quantitative determination was conducted with the use of bovine serum albumin, the concentration being 10 mg/ml when $OD_{278}$= 6.67.

In the purification operation, for samples to be treated with hydroxylapatite et seq, $OD_{215}$ and $OD_{225}$ were measured with a Kubota 150-02 photometer, and the value of $OD_{215}$ - $OD_{225}$ multiplied by 144 was given in terms of μg/ml.  According to this method, up to about 0.5 μg/ml of protein could be quantitatively determined without impairment of the sample [14], [15].

2) Antibacterial active protein and its induction:

The hemolymph of larvae two days after the injury was subjected to electrophoresis on a slab type 15% polyacrylamide gel (pH 4) under non-denaturing conditions, and the antibacterial activities of the bands formed were detected. The hemolymph was applied in a quantity of 4 μl (0.8 unit) and was made acidic with the addition of 1/2 quantity of 1M acetic acid. Electrophoresis was conducted at 200 V (constant voltage) from $\oplus$ to $\ominus$ for 6 hours. After completion of electrophoresis, a soft agar containing E. coli (K-12 SM$^r$) was overlaid on the gel and left to stand at 37°C overnight. As a result, three kinds of spots inhibited in bacterial colony formation were detected. Accordingly, the respective active substances were determined as the first component, the second component, and the third component, in the order of greater molecular weight. On the other hand, for the normal hemolymph, no such spot whatsoever was detected when an equivalent amount was applied, and the three kinds of the active components were all found to be induced by stimulation due to the body surface injury.

Furthermore, since these components were deactivated by trypsin treatment, they were found to be proteinaceous substances.

3) Separation and purification of antibacterial protein:

(1) Separation of the three components:

The starting material used for purification was the hemolymph of larvae two days after injury which may have the highest antibacterial activity. The hemolymph was collected from about 20,000 larvae and the operations were conducted with the use of this as the starting material.

After 20 ml of the hemolymph of larvae was diluted 5-fold with Buffer A, it was applied to a column of CM-cellulose. After application, the column was washed with ample amount of Buffer B, and the protein was eluted stepwise with Buffer E, when the $OD_{280}$ of the eluate became less than 0.01. Fractions each of 2 to 3 ml were collected, and a certain amount thereof was sampled by separation, diluted with buffer, and measured in a total amount of 200 µl. Activities were collected in fractions No. 33 - 40 with a yield of 97%, and the specific activity was increased to 100-fold.

Next, the fractions were left to stand in a boiling water bath for 10 minutes (heat treatment), cooled in ice, and centrifuged at 40,000 g (10 minutes, 4°C, Kubota RA-3). The supernatant was collected for examination of its activity, and it was found that the antibacterial substance in this fraction was thermally stable, the activity recovery was 95%, and the specific activity increase was about 5-fold. At the same time, it was also found that the heat treatment was useful as a measure for purification of the antibacterial substance. The thermal stability was also confirmed by the fact that the activity was not changed when an active amount (1.5 unit) which inhibited growth of bacteria by about 10% was sampled and subjected to heat treatment at 100°C for one hour.

By the two purifying measures as described above, 90% or more of the antibacterial protein was recovered, and the specific activity elevated to approximately 500-fold. The fractions were found to be stable for at least half a year when stored under refrigeration at -20°C. When the fractions were analyzed by polyacrylamide gel electrophoresis under non-denaturing conditions, it was indicated that three kinds of the active components were collected up to this purification stage.

The heat-treated supernatant fraction (60 ml) was concentrated to 2 ml by ultrafiltration (4°C, 4 $kg/cm^2$) by the use of a membrane (SPECTRA/POR UF DISCS φ 43 mm) with a cut-off molecular weight of 1,000. The activity recovery of the concentration operation was found to be 60 to 100%. The heat-treated supernatant fraction thus concentrated was applied to a Sephadex G-50 column (φ 1.5 x 60 cm). The Sephadex G-50 had previously been equilibrated with 5-fold column volume of Buffer S. Elution was carried out with Buffer S, and fractions each of 2 ml were collected. A certain amount was separated from each fraction and diluted to 200 μl with Buffer S before measurement. The activity was recovered by formation of two peaks at void and at around a molecular weight of 6 - 7 kilodaltons.

The active fraction on the higher molecular weight side is defined as G-1 fraction, and the active fraction on the lower molecular weight side as G-2 fraction. G-1 fraction was further subjected to gel filtration by high performance liquid chromatography (Waters, Protein Column I-125 φ 7.8 x 30 cm, two columns in series), whereby an active peak was recovered between the eluted positions of Rinderserum Albumin with molecular weight of 68 kilodalton and Hühnerei Albumin with molecular weight of 45 kilodalton, and its molecular weight was calculated to be about 54,000. G-2 fraction was measured with further dilution, whereby a substantially single active peak was obtained. Now, when the respective G-1 and G-2 fractions were analyzed by polyacrylamide gel electrophoresis under non-denaturing conditions, the first component was detected in G-1 fraction, and the second and third components in G-2 fraction.

From these results, the first component may be considered to be a protein with a molecular weight of about 54,000, while the second and third components may be considered to be proteins of 6 - 7 kilodaltons with similar molecular weights. The activity recovery of G-1 fraction was found to be 19% of the sample applied and that of G-2 fraction, 83%. Although the specific activity of G-1 fraction was lowered, the specific activity of G-2 fraction elevated to about 2-fold, with the total specific activity being substantially the same. Accordingly, this operation was found to be useful also as a purifying/separating measure.

The G-2 fraction containing the second and third components was diluted 5-fold with Buffer A, and this was applied to a CM-cellulose (column $\phi$ 2.0 x 4 cm). After application, the column was washed with 2-fold of column volume of Buffer A, which step was followed by stepwise elution of the proteins with Buffer S. When $OD_{280}$ became approximately zero, the proteins were further eluted stepwise with Buffer D. Fractions each of 2 to 4 ml were collected and measured. Activities were recovered respectively in eluted fractions with Buffer S and eluted fractions with Buffer D. The active fraction on the lower salt concentration side with Buffer B was defined as C-1 fraction and the active fraction on the higher concentration side with Buffer D was defined as C-2 fraction.

By analysis of polyacrylamide gel electrophoresis, it was indicated that the second component was recovered in the C-1 fraction and the third fraction in the C-2 fraction. The activity recovery of the C-1 fraction was found to be 2% based on the sample applied, and that of the C-2 fraction, 90%. Also, while the specific activity of the C-1 fraction was lowered, the specific activity of the C-2 fraction was elevated to about 6-fold, the total activity being elevated to about 1.6-fold. Thus, it was found at the same time that this operation is useful as a separating method.

After purification of all the three components, the three kinds of active fractions of G-1, C-1 and C-2 could thus be obtained by the two separation operations.

The results of analysis of the three kinds of active fractions thus obtained by polyacrylamide gel electrophoresis under non-denaturing conditions are shown in lanes G-1, C-1 and C-2 in Fig. 2. The lane H was obtained when the purified starting material of the hemolymph of larvae was applied. It is clear from the Figure 2 that, of the three kinds of active components found in the hemolymph:

the first component is separated into the G-1 fraction;

the second component into the C-1 fraction; and

the third component into the C-2 fraction; respectively.

Mutual contamination of respective activities was not detected.

From the above results, the method for separation of the three kinds of active components of the body fluid has been judged to be established. By the operations of purification and separation to this stage, the first component was purified to yield 12 %/specific activity 110-fold, the second component to yield 1 %/ specific activity 44-fold, and the third component to yield 45 %/specific activity 7,300-fold. As judged from activity recovery, the main active component in the body fluid may be considered to be the third component.

(2) Purification of the third component:

Subsequently, on the basis of the following standards of judgement, the above third component was subjected to purification:

(a) single band should be given by two kinds of electrophoretic systems of different principles; and

(b) antibacterial activities should correspond to respective proteins forming bands in respective electrophoreses.

First, 18 ml of the C-2 fraction containing the third component was diluted 10-fold with Buffer A, and this was then applied to a hydroxylapatite column ($\phi$ 2.0 x 2 cm). After application, the column was washed with 2-fold column volume of Buffer A, then with 40 ml of 50 mM phosphate buffer ($Na_2HPO_4/NaH_2PO_4$, pH 6), and proteins were eluted stepwise with 100 mM phosphate buffer, pH 6, whereby antibacterial activity was detected in fraction Nos. 45 through 59.  The activity recovery of the fractions was found to be 30% of the sample applied, and the specific activity elevated to 5-fold.

Since hydroxylapatite fractions were diluted in protein concentration and were inconvenient for analysis by electrophoresis, etc., they were concentrated with CM-cellulose.  Hydroxylapatite fractions were diluted with equivalent amount of deionized water and applied to a CM-cellulose column (φ 1.1 x 1 cm).  The CM-cellulose column had previously been equilibrated with 5-fold or more column volume of Buffer A.  After application, the column was washed with 4-fold column volume of Buffer A, and the proteins were eluted stepwise with Buffer E and fractionated into each 1 ml.  As a result, only in the second fraction of the eluted fractions with Buffer E, proteins were recovered (67 %).  The activity recovery of this fraction was found to be 65 %, substantially coinciding with the recovery of proteins.  Thus, the specific activity is not changed by this operation (purification being completed in the stage prior to this operation), and this operation may be said to be entirely a concentrating operation.  By this operation, 30 ml of the hydroxylapatite fraction was concentrated to 1 ml.

The third CM-cellulose fraction was analyzed by polyacrylamide gel electrophoresis under non-denaturing conditions.  Thus, 25 μl (1.2 μg) of the fraction was applied, and electrophoresis was carried out from ⊕ and ⊖ constantly at 200 V for 3 hours.  As a result, only a single band was detected by staining with Coomasie Brilliant Blue.  At the same time, for the CM-cellulose fraction subjected to electrophoresis, antibacterial activity was examined by overlaying soft agar containing E. coli on the gel.  As a result, corresponding to the positions of the bands formed by staining, spots inhibited in colony of E. coli were formed.  From these results, the CM-cellulose fraction was considered as the final product of purification, and the degree of purification of this preparation was examined as follows.

The CM-cellulose fraction was analyzed by the SDS polyacrylamide (15%) slab gel electrophoresis according to the Laemmli's system. Thus, 50 µl (2.4 µg) of the fraction was applied and electrophoresis was carried out constantly at 50V for about 12 hours. As a result, also in this system, one band was formed by staining with Coomasie Brilliant Blue. From the degree of migration of the marker, the molecular weight was estimated to be around 5,000.

Next, in order to examine whether the antibacterial activity corresponds to the single band obtained in the SDS-polyacrylamide gel electrophoresis or not, the CM-cellulose fraction was subjected to gel filtration with Sephadex G-50. Thus, 500 µl of the fraction was applied to a column of $\phi$ 1.1 x 60 cm and eluted with Buffer S. The column had previously been sufficiently equilibrated by washing with 5-fold or more of column volume of Buffer S. A substantially symmetric peak of protein was obtained with the fraction No.35 as the center. Then, in conformity with appearance and disappearance of this protein, the antibacterial activity was recovered. The recovery of the protein and that of activity were both about 30%. The three fractions of this peak were freeze-dried and then subjected to SDS-polyacrylamide (15 %) gel electrophoresis, whereby a single band with the same degree of migration before application to gel filtration was obtained. From this fact, it has been judged that the protein forming the single band in SDS-polyacrylamide gel electrophoresis has antibacterial activity. From the eluted positions of markers with various molecular weights, the molecular weight of the antibacterial protein in gel filtration was calculated to be about 5,000.

From the above results, it has been shown that the third CM-cellulose fraction gives single bands in electrophoretic systems of different electrophoretic systems, namely the polyacrylamide gel electrophoresis under non-denaturing conditions and the SDS-polyacrylamide gel electrophoresis, and antibacterial activity corresponded to each band. This satisfies the standards of judgement for completion of purification, and thus it has been judged that the purification of the antibacterially active third component in the hemolymph of larvae has been completed.

From the results as described above, three kinds of antibacterial components were confirmed by use of 130 ml of the hemolymph collected from about 20,000 larvae two days after injury to their body walls as the starting material. Particularly, about 70 µg of the third component was obtained at the stage of hydroxylapatite fractionation. The specific activity of this component was found to be elevated to about 30,000-fold, with the activity recovery being 14 %. From the specific activity 27,000 units/mg of the final preparation, 0.1 µg of the third component was calculated to have a lethal activity against $\underline{E}$. $\underline{coli}$ of the order of $10^7$. This may be said to be a potent antibacterial activity, as compared with antibiotics in general.

The purification operations described above are summarized in the following Table.

Table 1. Purification of antibacterial substance

| Purification stage | Total protein (mg) | Total activity (unit x $10^{-2}$) | Specific activity (unit/mg) | Activity yield (%) |
|---|---|---|---|---|
| Hemolymph | 18100 | 136 | 0.75 | 100 |
| First CM-cellulose | 156 | 132 | 82 | 97 |
| Heat treatment | 31 | 126 | 410 | 93 |
| Concentration | - | 84 | - | 62 |
| Sephadex G-50, G-1 | 19 | 16 | 85 | 12 |
| G-2 | 8.2 | 70 | 850 | 51 |
| Second CM-cellulose C-1 | 3.4 | 1.1 | 33 | 1 |
| C-2 | 1.1 | 61 | 5,500 | 45 |
| Hydroxylapatite | 0.07 | 19 | 27,000 | 14 |
| Third CM-cellulose | 0.047 | 13 | 27,000 | 9 |

4)  Obtaining antibacterial polypeptide of the present invention:

The third component purified (hereinafter called "sarcotoxin I") was fractionated by use of HPLC reversed phase chromatography [Synchropack RPP-C18 column (Gilson Co.)] to obtain three peaks of sarcotoxin IA, IB and IC (Fig. 1).  The conditions for HPLC are shown below.

Solution A: 0.05% trifluoroacetic acid/water

Solution B: 0.05% trifluoroacetic acid/99% acetonitrile

Gradient:  linear gradient of 15% of Solution B in Solution A, 50% of Solution B in Solution A;

Flow rate:  2 ml/min.

Thus, as is apparent from Fig. 1, between 30 and 35% of Solution B, sarcotoxin IA, IB and IC were separated.

The sarcotoxin IA, IB and IC obtained were found to have the amino acid sequences as shown below by the gas phase protein sequencer (Applied Biosystems 470A Protein Sequencer).

```
                      1                   5
       IA  H₂N-Gly-Trp-Leu-Lys-Lys-Ile-Gly-
       IB                      -Lys-
       IC                      -Arg-

                     10                  15
           Lys-Lys-Ile-Glu-Arg-Val-Gly-Gln

                           20
           -His-Thr-Arg-Asp-Ala-Thr-Ile-

                 25                  30
           Gln-Gly-Leu-Gly-Ile-Ala-Gln-Gln
                 -Val-Ile-    -Val-
                 -Val-Leu-    -Ile-

                                35
           -Ala-Ala-Asn-Val-Ala-Ala-Thr-

                 39
           Ala-Arg-COOH
```

5) Properties of antibacterial polypeptide:

Sarcotoxin IA, IB and IC obtained in the above operation have the same antibacterial spectrum as the third component (sarcotoxin I) in the Laid-Open Patent Publication _supra_. The method for measuring the antibacterial spectrum of the third component and the results obtained are shown below.

Two units of sample were employed. After sterilization with ether, ether was evaporated off, and the residue was placed in a sterilized plastic laboratory dish. Then 9 ml of Mueller-Hinton medium sterilized and liquefied in an autoclave (ca. 60°C) was poured thereon (total amount 10 ml), which step was followed immediately by stirring for mixing with the sample, and the mixture was left to stand at 30°C to solidify the medium. On this medium, 56 kinds of bacterial liquors previously cultured for two days each of about $10^6$ cells were inoculated at constant intervals. After the inoculated medium was left to stand at 37°C overnight, colony formations of respective bacteria were observed. The case of the same extent of colony as Control was judged as +, the case of markedly smaller colony than control as $\pm$, and the case of no colony formed as −. However, for only M.607 bacterium, the same judgement was made after cultivation for two days. The Control was measured for Buffer S of the same volume as a sample containing 0.2 % bovine serum albumin.

The results are summarized in the following Table.

## Table 2. <u>Antibacterial spectrum</u>

(+) Colony formation: present
(-)    "        "    : absent

| Bacteria tested | Third component | Control |
|---|---|---|
| Streptomyces aureus 209P | + | + |
| "            Smith | + | + |
| "            Ap01 | + | + |
| Micrococcus flavus FDA16 (ATCC 10240a) | - | + |
| Sarcina lutea PCI 1001 (ATCC 9341) | + | + |
| Bacillus anthracis | + | + |
| Bacillus subtilis PCI 219 (ATCC 6633) | + | + |
| "            NRRLB-558 (ATCC 9466) | + | + |
| Bacillus cereus ATCC10702 | + | + |
| Bacillus megaterium APF | - | + |
| Corynebacterium bovis 1810 | - | + |
| Escherichia coli NIHJ | - | + |
| "        K-12 | - | + |
| "        K-12,R5 | - | + |
| "        K-12,R388 | - | + |
| "        K-12,J5R11-2 | - | + |
| "        K-12,ML1629 | - | + |
| "        K-12,ML1630 | - | + |
| "        K-12,ML1410 | - | + |
| "        K-12,ML1410 R81 | - | + |
| "        K-12,LA290 R55 | - | + |
| "        K-12,LA290 R56 | - | + |
| "        K-12,LA290 R64 | - | + |
| "        W 677 | - | + |
| "        JR66/W677 | - | + |
| "        K-12,C600 R135 | - | + |
| "        JR255 | - | + |

24

Table 2 (continued)

| Bacteria tested | Third component | Control |
|---|---|---|
| Mycobacterium smegmatis (ATCC 607) | + | + |
| Klebsiella pneumoniae PCI602 (ATCC 10031) | + | + |
| "     -22 # 3038 | + | + |
| Shigella dysenteriae JS11910 | ± | + |
| Shigella flexneri 46 JS11811 | + | + |
| Shigella sonnei JS11746 | − | + |
| Salmonella typhi T-63 | + | + |
| Salmonella enteritidis 1891 | + | + |
| Proteus vulgaris OX19 | − | + |
| Proteus rettgeri GN466 | + | + |
| "         GN466 | − | + |
| Serratia marcescens | + | + |
| Serratia SOU(594) | + | + |
| Serratia 4 (755) | + | + |
| Providencia Pv16 (641) | − | + |
| "     2991 (741) | − | + |
| Pseudomonas aeruginosa A3 | + | + |
| "              NO12 | + | + |
| "              H9 | + | + |
| "              H11 | + | + |
| "              TI-13 | + | + |
| "              GN315 | + | + |
| "              99 | + | + |
| "              B-13 (534) | + | + |
| "              21-75 | + | + |
| "              PST-1 | + | + |
| "              ROS134/PO21 | + | + |
| "              K-Ps 102 | + | + |
| Pseudomonas maltsphilia GN707 | + | + |

25

As a result, the third component was found to have potent antibacterial activity against E. coli and bacteria similar thereto. Also, as for E. coli K-12, since antibacterial activities were exhibited against various drug resistant strains (aminoglycoside type antibiotic), the action of this substance suggests that it is of a type different from the existing aminoglycoside type antibiotics.

Generally speaking, many antibacterial substances are more effective against Gram (+) bacteria. Also in this sense, it is of interest in considering the mechanism of the antibacterial action that the substance of the present invention is effective against Gram (-) bacteria and can also act partially on Gram (+) bacteria.

"Sephadex" used herein is a bead-like product of dextran cross-linked with epichlorohydrin into a three-dimensional structure.

"CM-cellulose" used herein is a product of fibrous cellulose carboxymethylated.

"Hydroxylapatite" is a product of calcium phosphate having a formula: $Ca_{10}(PO_4)_6(OH)_2$

References:

(1)  Nature, 222, 695 (1969).

(2)  Journal of Invertebrate Pathology, 32, 171 (1978).

(3)  Journal of Insect Physiology, 14, 1025 (1968).

(4)  European Journal of Biochemistry, 106, 7 (1980).

(5)  Journal of Insect Physiology, 23, 1169 (1977).

(6)  The Journal of Biological Chemistry, 255, 2919 (1980).

(7)  The Journal of Biological Chemistry, 256, 7087 (1981).

(8)  Nature, 279, 725,728 (1979).

(9)  Biochemistry, 20, 5973 (1981).

(10) 101st Annual Meeting of Pharmacological Society of Japan (1981).

(11) Nature, 227, 680 (1970).

(12) Biochemistry, 10, 2606 (1971).

(13) J. of Biochemistry, 193, 265 (1951).

(14) Nordisk Medicine, 58, 1488 (1957).

(15) J. of Biochemistry, 46, 517 (1959).

27

WHAT IS CLAIMED IS:

1.      An antibacterial polypeptide represented by the amino acid sequence:

$H_2$N-Gly-Trp-Leu-$X_1$-Lys-Ile-Gly-Lys-
Lys-Ile-Glu-Arg-Val-Gly-Gln-His-
Thr-Arg-Asp-Ala-Thr-Ile-Gln-$X_2$-$X_3$
-Gly-$X_4$-Ala-Gln-Gln-Ala-Ala-Asn-
Val-Ala-Ala-Thr-Ala-Arg-COOH

wherein: $X_1$ represents Lys or Arg; $X_2$, Gly or Val; $X_3$, Leu or Ile; and $X_4$, Ile or Val.

2.      A polypeptide according to Claim 1, wherein $X_1$, $X_2$, $X_3$, and $X_4$ are Lys, Gly, Leu, and Ile, respectively.

3.      A polypeptide according to Claim 1, wherein $X_1$, $X_2$, $X_3$, and $X_4$ are Lys, Val, Ile, and Val, respectively.

4.      A polypeptide according to Claim 1, wherein $X_1$, $X_2$, $X_3$, and $X_4$ are Arg, Val, Leu, and Ile, respectively.

5.      A process for producing an antibacterial polypeptide, which comprises imparting injury to the body wall of a larva of an insect belonging to the order Diptera or Hymenoptera, obtaining the hemolymph from the larva thus injured, removing hemocytes and fatty components from said hemolymph, and then obtaining, from the thus purified hemolymph, a polypeptide represented by the amino-acid sequence formula

$H_2$N-Gly-Trp-Leu-$X_1$-Lys-Ile-Gly-Lys-
Lys-Ile-Glu-Arg-Val-Gly-Gln-His-
Thr-Arg-Asp-Ala-Thr-Ile-Gln-$X_2$-$X_3$
-Gly-$X_4$-Ala-Gln-Gln-Ala-Ala-Asn-
Val-Ala-Ala-Thr-Ala-Arg-COOH

wherein: $X_1$ represents Lys or Arg; $X_2$, Gly or Val; $X_3$, Leu or Ile; and $X_4$, Ile or Val.

6.      A process according to Claim 5, wherein $X_1$, $X_2$, $X_3$ and $X_4$ are Lys, Gly, Leu and Ile, respectively.

7.      A process according to Claim 5, wherein $X_1$, $X_2$, $X_3$ and $X_4$ are Lys, Val, Ile and Val, respectively.

8.      A process according to Claim 5, wherein $X_1$, $X_2$, $X_3$ and $X_4$ are Arg, Val, Leu and Ile, respectively.

9.      A process according to any of Claims 5 to 8, wherein the insect belonging to the order Diptera or Hymenoptera is <u>Sarcophaga peregrina</u>.

10.     A process according to any of Claims 5 to 9, wherein the larva is a third-instar larva.

11.     A process according to any of Claims 5 to 10, wherein the hemolymph of the larva is obtained 0 to 5 days after injury of the body wall.

12.     A process according to any of Claims 5 to 11, wherein the step of obtaining the objective peptide comprises subjecting the purified hemolymph to chromatography to obtain a fraction which has the highest antibacterial activity among the fractions obtained by the chromatography, and subjecting the fraction to HPLC reversed phase chromatography, and eluting the fractions.

13.     A process according to any of Claims 5 to 12, wherein the step of obtaining the objective polypeptide includes the operation of subjecting the materials to heat treatment at 80 to 120°C.

14.     An edible antibacterial agent comprising, as the effective ingredient, a polypeptide represented by the amino acid sequence formula

H$_2$N-Gly-Trp-Leu-X$_1$-Lys-Ile-Gly-Lys-
    Lys-Ile-Glu-Arg-Val-Gly-Gln-His-
    Thr-Arg-Asp-Ala-Thr-Ile-Gln-X$_2$-X$_3$
    -Gly-X$_4$-Ala-Gln-Gln-Ala-Ala-Asn-
    Val-Ala-Ala-Thr-Ala-Arg-COOH

wherein: X$_1$ represents Lys or Arg; X$_2$, Gly or Val; X$_3$, Leu or Ile; and X$_4$, Ile or Val.

15.    An edible antibacterial agent according to Claim 14, wherein X$_1$, X$_2$, X$_3$ and X$_4$ are Lys, Gly, Leu and Ile, respectively.

16.    An edible antibacterial agent according to Claim 14, wherein X$_1$, X$_2$, X$_3$ and X$_4$ are Lys, Val, Ile and Val, respectively.

17.    An edible antibacterial agent according to Claim 14, wherein X$_1$, X$_2$, X$_3$ and X$_4$ are Arg, Val, Leu and Ile, respectively.

FIG. I

FIG. 2